# EUROPEAN PATENT APPLICATION

(11) **EP 2 442 277 A2**
(43) Date of publication of application: **18.04.2012**
(21) Application number: 11184725.7
(22) Date of filing: 11.10.2011
(51) Int. Cl.: G06T 7/00

(54) **Diagnosis assisting apparatus, diagnosis assisting program, and diagnosis assisting method**

(30) Priority: 12.10.2010 JP 2010229300
(71) Applicant: Fujifilm Corporation, Minato-ku Tokyo (JP)
(72) Inventor: Mizuno, Osamu, Tokyo (JP)
(74) Representative: Klunker . Schmitt-Nilson . Hirsch

(57) **Abstract**

Assisting diagnosis performed by a doctor by calculating a first index value representing a direction and magnitude of motion of each portion of the heart for a three-dimensional anatomical image at a given phase of a three-dimensional anatomical image group representing a heart at each phase with respect to a further phase, calculating a second index value that serves as a diagnostic indicator of each portion of the heart from the three-dimensional anatomical image (7) at the given phase or the three-dimensional anatomical image group, generating a two-dimensional image (I) of the entire heart in which the second index value of each portion of the heart is disposed in a planar fashion, and displaying a mark (Vᵢ') representing the first index value superimposed on the generated two-dimensional image (I) in a manner that allows the direction of motion of the first index value to be visually recognized.

## Description

### BACKGROUND OF THE INVENTION

### Field of the Invention

The present invention relates to an apparatus, method, and computer program for assisting diagnosis by displaying cardiac motion in a two-dimensional image representing cardiac function calculated from three-dimensional image data.

### Description of the Related Art

As a device for assisting a radiologist in performing image diagnosis, an apparatus that analyzes a state or motion of an organ of a subject based on three-dimensional data obtained by imaging the subject and displays analysis results on a screen in a manner appropriate for diagnosis is provided. As for the function to analyze cardiac beat motion, a function that calculates evaluation values of cardiac function (myocardial wall motion, wall thickness variation, and the like) based on time series three-dimensional data is known. A plurality of cross-sections perpendicular to an axis passing through the cardiac base (where a blood vessel is connected on the upper side of a heart) and the cardiac apex (pointed portion on the lower side) is set and these evaluation values are calculated for each cross-section. Typically, the calculated evaluation values are displayed three-dimensionally according to cardiac shape. In the mean time, as for the method of displaying analysis results two-dimensionally, a bull's eye image in which evaluation values of each cross-section are disposed on the circumferences of concentric circles having different radii is known.

For example, Japanese Unexamined Patent Publication No. 2002-306483 discloses a method that extracts information of a contour of an organ from each of a plurality of cross-sectional images with respect to a plurality of phases, calculates a three-dimensional contour of the organ from the information of the plurality of extracted contours, calculates information of motion of the three-dimensional contour based on a travel distance between corresponding apexes on the three-dimensional contour of each phase, superimposes a mesh display, in which points constituting a contour are connected by a straight line, on three-dimensional contours displayed in different colors based on the information of motion of each apex.

U.S. Patent Application Publication No.20090043200 discloses a method that displays spatiotemporal propagation of cardiac mechanical motion by extracting, with respect to a plurality of phases, a region in which cardiac mechanical motion is not less than a predetermined value and displaying the extracted region in a bull' s eye image.

The method described in Japanese Unexamined Patent Publication No. 2002-306483, however, poses problems that it is necessary to display and confirm three-dimensional contours of a heart from a plurality of directions in order to obtain motion information of the entire heart, and it is necessary to display and compare a plurality of three-dimensional contours of different phases in order to confirm the trajectory of motion of a specific apex of the heart, thereby requiring time for understanding cardiac motion.

The method described in U.S. Patent Application Publication No. 20090043200 may confirm a region in which cardiac mechanical motion is not less than a predetermined value with respect to each phase, but it is difficult to understand which direction each region constituting the heart moves according to the phase change.

In view of the circumstances described above, it is an object of the present invention to display index values representing function of the entire heart and motion of each cardiac region in an easily understandable manner, thereby assisting a doctor in diagnosis.

### SUMMARY OF THE INVENTION

A diagnosis assisting apparatus of the present invention is an apparatus, including:
an image obtaining means for obtaining a three-dimensional anatomical image group that includes three-dimensional anatomical images, each three-dimensionally representing a structure of a heart, obtained by imaging the heart at each phase;
a first index value calculation means for calculating a first index value representing a direction and magnitude of motion of each portion of the heart for a three-dimensional anatomical image at a given phase of the three-dimensional anatomical image group with respect to a further phase;
a second index value calculation means for calculating a second index value that serves as a diagnostic indicator of each portion of the heart from the three-dimensional anatomical image at the given phase or the three-dimensional anatomical image group;
a two-dimensional image generation means for generating a two-dimensional image of the entire heart in which the second index value of each portion of the heart is disposed in a planar fashion; and
a display control means for displaying the generated two-dimensional image on a display device and superimposing a mark representing the first index value calculated by the first index value calculation means on the displayed two-dimensional image in a manner that allows the direction of motion of the first index value to be visually recognized.

A diagnosis assisting method of the present invention is a computer-implemented method, including the steps of:
obtaining a three-dimensional anatomical image group that includes three-dimensional anatomical images, each three-dimensionally representing a structure of a heart, obtained by imaging the heart at each phase;
calculating a first index value representing a direction and magnitude of motion of each portion of the heart for a three-dimensional anatomical image at a given phase of the three-dimensional anatomical image group with respect to a further phase;
calculating a second index value that serves as a diagnostic indicator of each portion of the heart from the three-dimensional anatomical image at the given phase or the three-dimensional anatomical image group;
generating a two-dimensional image of the entire heart in which the second index value of each portion of the heart is disposed in a planar fashion; and
displaying the generated two-dimensional image on a display device and superimposing a mark representing the first index value calculated by the first index value calculation means on the displayed two-dimensional image in a manner that allows the direction of motion of the first index value to be visually recognized.

A diagnosis assisting program of the present invention is a program for causing a computer to function as:
an image obtaining means for obtaining a three-dimensional anatomical image group that includes three-dimensional anatomical images, each three-dimensionally representing a structure of a heart, obtained by imaging the heart at each phase;
a first index value calculation means for calculating a first index value representing a direction and magnitude of motion of each portion of the heart for a three-dimensional anatomical image at a given phase of the three-dimensional anatomical image group with respect to a further phase;
a second index value calculation means for calculating a second index value that serves as a diagnostic indicator of each portion of the heart from the three-dimensional anatomical image at the given phase or the three-dimensional anatomical image group;
a two-dimensional image generation means for generating a two-dimensional image of the entire heart in which the second index value of each portion of the heart is disposed in a planar fashion; and
a display control means for displaying the generated two-dimensional image on a display device and superimposing a mark representing the first index value calculated by the first index value calculation means on the displayed two-dimensional image in a manner that allows the direction of motion of the first index value to be visually recognized.

The image obtaining means of the diagnosis assisting apparatus of the present invention is a means that obtains a three-dimensional anatomical image three-dimensionally representing a structure of an organ, but it may be a means that further obtains a three-dimensional functional image in which evaluation values for evaluating function of the organ are disposed three-dimensionally according to the shape of the organ. The image obtaining means obtains these images from a built-in memory or storage of a computer that functions as the diagnosis assisting apparatus or from an external storage coupled to the computer directly or via a network. The three-dimensional functional image is a volume data with evaluation values (e.g., values representing motion or physiological reaction of the organ) for evaluating as to whether or not the organ is functioning normally as voxel data. The three-dimensional anatomical image is a volume data with values representing an anatomical structure of an organ as voxel data.

The first index value may include any type of index value that represents a direction and magnitude of motion of each cardiac wall portion between a three-dimensional anatomical image at a give phase and a three-dimensional anatomical image at a further phase. Typical examples are cardiac wall displacement, cardiac wall movement speed, variance in cardiac wall movement speed (acceleration) between specific phases, such as from the end-diastole to end-systole. Further, the first index value may be a value of data constituting a three-dimensional functional image or a bull's eye image, or a value obtained as a result of certain analysis performed on the value of data. The value obtained as a result of certain analysis performed on the value of data may include, for example, an average value (including weighted average or the like), maximum value, or minimum value of each data of movement speed and acceleration of cardiac wall between specific phases, such as from the end-diastole to the end-systole. Further, the first index may be represented, for example, by a ratio of radius from the center of the heart, cardiac wall thickness, or the like. Displacement, movement speed, acceleration, and the like of each cardiac wall may be divided into a component in a direction radially extending from the center of the heart to outside and a component on a plane perpendicular to the direction radially extending from the center of the heart to outside and the component on the plane perpendicular to the direction radially extending from the center of the heart to outside may be used as the first index value.

The "second index value that serves as a diagnostic indicator" may be anything as long as it is useful for diagnosis. The second index value may be a value of data constituting a three-dimensional functional image or a bull's eye image, or a value obtained as a result of certain analysis performed on the value of data. For example, a value obtained by the calculation process for calculating an average value (including weighted average or the like), maximum value, or minimum value of data constituting a three-dimensional anatomical image or a three-dimensional functional image. Specific examples of second index values may include those used in bull's eye image display, such as a radius of each cardiac portion, ejection fraction, cardiac wall thickness, pixel value of cardiac wall (which may include an average of pixel values of each cardiac portion). Further, the second index value may be the same as the first index value, A component of displacement, movement speed, acceleration, and the like of each cardiac wall in a direction radially extending from the center of the heart to outside may be used as the second index value while a component on a plane perpendicular to the direction radially extending from the center of the heart to outside may be used as the first index value.

The "two-dimensional image of the entire heart in which the second index value of each portion of the heart is disposed in a planar fashion" may include, for example, a bull' s eye image based on the second index value of each cardiac portion and an image of the entire heart in which the second index value of each cardiac portion is disposed in rectangle. The image in which the second index value of each cardiac portion is disposed in rectangle may be generated using, for example, a method described in "Three-Dimensional Display of Positron Emission Tomography of the Heart", The Journal of Nuclear Medicine 29, pp.530-537, 1988, and projecting the second index value of each cardiac portion in a cylindrical shape.

The image obtaining means obtains Nₜ (integer not less than 2) three-dimensional anatomical images representing structures of a heart at different phases. Since how many three-dimensional anatomical images of different phases are required depends on the type of the first index, the image obtaining means obtains the number of three-dimensional anatomical images of different phases required for calculating the first index value. For example, in the case where the displacement or speed of each cardiac portion is calculated, the image obtaining means obtains three-dimensional anatomical images of at least two phases, while in the case where acceleration of each cardiac portion is calculated, the image obtaining means obtains three-dimensional anatomical images of at least three different phases.

Further, in the diagnosis assisting apparatus of the present invention, the first index value calculation means may be a means that obtains the first index value at each of a plurality of phase of the heart and the display control means may be a means that displays the mark at each phase in a time series manner. For example, in a typical cardiac examination, a plurality of three-dimensional anatomical images at different phases is obtained and a plurality of three-dimensional anatomical images is stored per each examination. In the case where three-dimensional anatomical images of an organ at times T₁, T₂, T_{Nt} are available, the first index value calculation means may extract the first index value for a three-dimensional anatomical image at a phase of time Tᵢ+1 with respect to a three-dimensional anatomical image at a phase of time Tᵢ, with respect to each group of Nₜ three-dimensional anatomical images, and the first index value at each phase may be displayed in a time series manner.

In the diagnosis assisting apparatus of the present invention, the image obtaining means may be a means that further obtains a three-dimensional functional image in which evaluation values for evaluating function of the heart are disposed three-dimensionally according to the shape of the heart, and the second index value calculation means may be a means that calculates the second index value that serves as a diagnostic indicator from the three-dimensional functional image instead of at least either one of the three-dimensional anatomical image at the given phase and the three-dimensional anatomical image group.

Further, in the diagnosis assisting apparatus of the present invention, it is preferable that the display control means is a means that displays the mark representing the first index value at each of a plurality of points disposed regularly in the two-dimensional image. For example, the display control means may display the mark representing the first index value at each of a plurality of points disposed in a grid pattern in the two-dimensional image. In the case where the two-dimensional image is a bull's eye image, the display control means may display the mark representing the first index value at each of a plurality of points disposed at an equal interval on circumferences of concentric circles of different radii.

Further, "superimposing ------ in a manner that allows the direction of motion of the first index value to be visually recognized" may be realized by displaying the first index value in any manner as long as it allows the direction of motion to be visually recognized. For example, the display control means may use any mark as long as it represents a direction of cardiac motion as the mark representing the first index value. As an example, the display control means may use an arrow as the mark, and it is preferable that the arrow is displayed in a direction corresponding to the direction of motion of each cardiac portion with a length or width corresponding to the magnitude of the motion of each cardiac portion.

Further, the display control means may be a means that displays a trajectory of each of the plurality of points in the two-dimensional image due to a change in phase of the heart as the mark representing the first index value.

Still further, the display control means may be a means that uses each of a plurality of points disposed in the two-dimensional image so as to form a regular grid and shifted based on a magnitude and direction of the first index value at each point as the mark representing the first index value.

Further, a different color may be disposed and displayed on each point of the mark representing the first index value according to the direction or magnitude of the motion of each cardiac portion.

According to the diagnosis assisting apparatus, method, and program of the present invention, a mark representing a first index value is superimposed on a two-dimensional image of the entire heart in which the second index value of each portion of the heart is disposed in a planar fashion in a manner that allows the direction of motion of the first index value to be visually recognized, so that index values that serve as diagnostic indicators of the entire heart and the direction of motion of each cardiac portion may be displayed simultaneously on the displayed image. This allows the doctor or radiologist to confirm both the index values serving as diagnostic indicators of the entire heart and direction of motion of each cardiac portion on the displayed image, whereby the index values serving as diagnostic indicators of the entire heart and direction of motion of each cardiac portion maybe easily understood.

In the diagnosis assisting apparatus of the present invention, in the case where the image obtaining means is a means that further obtains a three-dimensional functional image in which evaluation values for evaluating function of the heart are disposed three-dimensionally according to the shape of the heart, and the second index value calculation means is a means that calculates the second index value that serves as a diagnostic indicator from the three-dimensional functional image instead of at least either one of the three-dimensional anatomical image at the given phase and the three-dimensional anatomical image group, index values that serve as diagnostic indicators of the entire heart and the direction of motion of each cardiac portion may be displayed simultaneously on the displayed image. This allows the doctor or radiologist to confirm both the index values serving as diagnostic indicators of the entire heart and direction of motion of each cardiac portion on the displayed image, whereby the index values serving as diagnostic indicators of the entire heart and direction of motion of each cardiac portion may be easily understood.

In the diagnosis assisting apparatus of the present invention, in the case where the first index value calculation means is a means that obtains the first index value for three-dimensional anatomical images at a plurality of phases of the heart, and the display control means is a means that displays the mark at each of the plurality of phases in a time series manner, index values that serve as diagnostic indicators of the entire heart and the direction of motion of each cardiac portion may be displayed simultaneously on the displayed image, and further the cardiac motion that varies with each phase may be easily understood in a time series manner.

Further, in the diagnosis assisting apparatus of the present invention, in the case where the display control means is a means that displays the mark representing the first index value at each of a plurality of points disposed regularly in the two-dimensional image, on the displayed image, index values that serve as diagnostic indicators of the entire heart and the direction of motion of each cardiac portion may be displayed simultaneously on the displayed image, and further the direction of motion of each cardiac portion may be understood more easily.

Still further, in the case where the display control means uses an arrow as the mark representing the first index value, it is easy to understand the direction of motion of each cardiac portion.

Further, in the case where the display control means is a means that displays a trajectory of each of the plurality of points in the two-dimensional image due to a change in phase of the heart as the mark representing the first index value, it is easy to understand the direction of motion of each cardiac portion.

Still further, in the case where the display control means is a means that uses each of a plurality of points disposed in the two-dimensional image so as to form a regular grid and shifted based on a magnitude and direction of the first index value at each point as the mark representing the first index value, it is easy to understand the direction of motion of each cardiac portion.

Further, in the diagnosis assisting apparatus of the present invention, in the case where the two-dimensional image generation means is a means that generates a bull's eye image as the two-dimensional image, index values that serve as diagnostic indicators of the entire heart and the direction of motion of each cardiac portion may be displayed simultaneously in the displayed bull's eye image. This allows the doctor or radiologist to confirm both the index values serving as diagnostic indicators of the entire heart and direction of motion of each cardiac portion in the displayed bull's eye image, whereby the index values serving as diagnostic indicators of the entire heart and direction of motion of each cardiac portion may be easily understood.

### BRIEF DESCRIPTION OF THE DRAWINGS

Figure 1 is a schematic diagram of a diagnosis assisting apparatus according to an embodiment of the present invention, illustrating a schematic configuration thereof.
Figure 2 is a flowchart illustrating a flow of processing performed by the diagnosis assisting apparatus.
Figure 3 illustrates a long axis and cross-sections set in a cardiac function analysis process.
Figure 4 illustrates an example evaluation value calculated in the cardiac function analysis process.
Figure 5 illustrates an example bull's eye image.
Figure 6 illustrates a calculation method of a first index value.
Figure 7 illustrates an example bull' s eye image with an arrow representing a first index value of the present embodiment superimposed thereon.
Figure 8 illustrates an example bull' s eye image with a grid representing a first index value of the present embodiment superimposed thereon.
Figure 9 illustrates an example bull's eye image with a trajectory representing a first index value of the present embodiment superimposed thereon.
Figure 10 illustrates an example modification of the present embodiment

### DESCRIPTION OF THE PREFERRED EMBODIMENTS

Hereinafter, embodiments of a diagnosis assisting apparatus, diagnosis assisting program, and diagnosis assisting method of the present invention will be described in detail with reference to the accompanying drawings.

In each embodiment to be described herein below, diagnosis assisting apparatus 1 includes one computer on which a diagnosis assisting program of each embodiment is installed. The computer may be a workstation or a personal computer directly operated by a doctor who performs diagnosis, or it may be a server computer linked to the workstation or personal computer via a network. The diagnosis assisting program is stored in a recording medium, such as DVD, CD-ROM, or the like, and distributed, which is then installed on the computer from the recording medium. Alternatively, the program is stored in a storage device of a server computer linked to a network or in a network storage in a manner accessible from outside, then the program is down loaded and installed on the computer used by the doctor in response to a request.

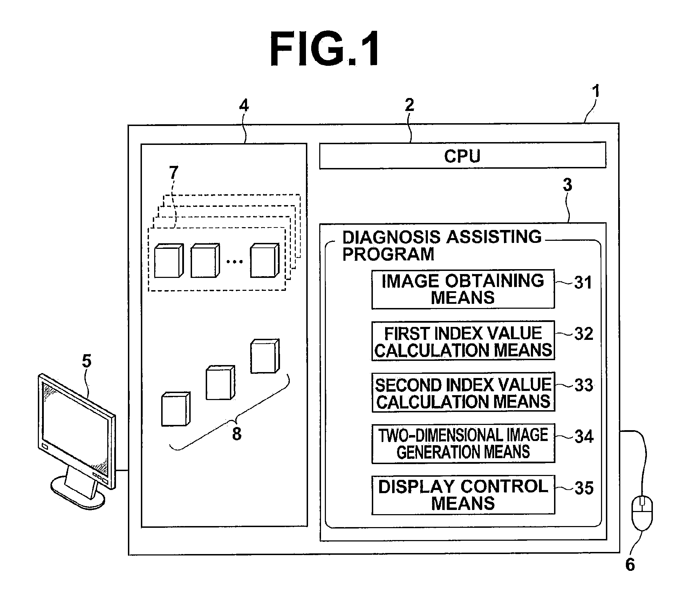

Figure 1 is a diagram of a diagnosis assisting apparatus realized by installing diagnosis assisting program on a workstation, illustrating a schematic configuration thereof. As illustrated in Figure 1, diagnosis assisting apparatus 1 includes CPU 2, memory 3, and storage 4 as a typical workstation configuration. Further, display 5 and input device 6, such as a mouse, are connected to diagnosis assisting apparatus 1.

Storage 4 includes, as three-dimensional anatomical images 7, volume data reconstructed from slice data outputted from a CT (Computed Tomography) system or an MRI (Magnetic Resonance Imaging) system, volume data outputted from an MS (Multi-Slice) CT system or a cone-beam CT system, and the like. Each volume data constitutes a series of volume data group obtained by imaging a subject Nₜ times at a predetermined time interval ΔT. Here, Nₜ is an integer not less than 2.

Storage 4 further includes, as three-dimensional functional images 8, SPECT images outputted from a SPECT (Single Photon Emission Computed Tomography) system, functional images generated by analyzing volume data outputted from an MSCT system, and the like. As described later, the analysis function of volume data (cardiac function analysis function) is provided as one of the functions of the diagnosis assisting program in the present embodiment.

Memory 3 includes the diagnosis assisting program and data to be referenced by the diagnosis assisting program (processing parameters and the like) . The diagnosis assisting program defines, as the processing to be performed by CPU2, an image obtaining process, a first index calculation process, a second index calculation process, a two-dimensional image generation process, and a display control process. When each of the processes described above is performed by CPU 2 according to the prescription of the program, the general purpose workstation functions as image obtaining means 31, first index value calculation means 32, second index value calculation means 33, two-dimensional image generation means 34, and display control means 35.

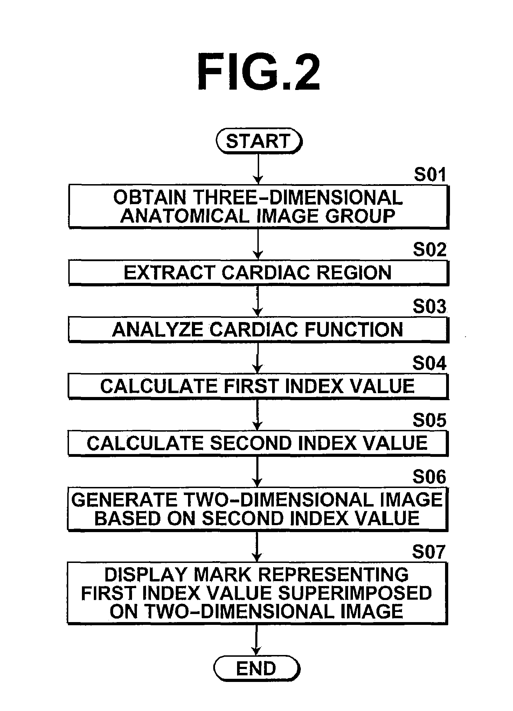

Figure 2 is a flowchart illustrating a flow of a diagnosis assisting method performed by the diagnosis assisting program of the present embodiment. Hereinafter, the diagnosis assisting method of the present embodiment will be described with reference to Figure 2.

When detects a selection of cardiac function diagnosis assisting function in a selection menu, diagnosis assisting apparatus 1 displays a list of subject IDs. When detects a user selection operation, image obtaining means 31 of diagnosis assisting apparatus 1, loads a group of three-dimensional anatomical images 7 in memory 3, as an image file related to the selected subject (S01).

In a typical cardiac examination, a plurality of three-dimensional anatomical images 7 is obtained at different phases and a plurality of three-dimensional anatomical images 7 is stored per each examination. In the present embodiment, image obtaining means 31 obtains Nₜ (integer not less than 2) three-dimensional anatomical images .7 representing structures of a heart at different phases. Here, a series of three-dimensional anatomical images taken at time T1, T₂, -----, T_{Nt} are obtained by imaging the subject at a constant time interval ΔT.

Further, when detects a user selection operation, image obtaining means 31 may obtain all of image files related to the selected subject. For example, in the case where a plurality of types of examinations (e.g., CT and SPECT examinations) have been performed and, as a result, a three-dimensional anatomical image 7 and a three-dimensional functional image 8 have been stored in storage 4, the two types of images may be loaded in memory 3.

First index value calculation means 32 performs a cardiac region extraction process on each three-dimensional anatomical image 7 constituting the obtained group of three-dimensional anatomical images 7 (S02).

For an overlapping process between the first and second index value calculation means, such as the case where the same three-dimensional anatomical image is used by the first and second index value calculation means, the process maybe performed by either one of them and the other of them may use the result. Here, in the case where a three-dimensional anatomical image 7 used for calculating the first index value and a three-dimensional anatomical image 7 used for calculating the second index value are different or a three-dimensional functional image 8 is further used, second index value calculation means 33 performs the cardiac region extraction process on the three-dimensional anatomical image or three-dimensional anatomical image group, or three-dimensional functional image 8.

A three-dimensional anatomical image of which phase is required is dependent upon the type of second index. Therefore, in the case where three-dimensional images of different phases are required for calculating the second index, the cardiac region extraction process is performed on the three-dimensional anatomical image group, while in the case where a three-dimensional image at only one phase is required, the cardiac region extraction process may be performed only on the three-dimensional image.

In the cardiac region extraction process, a cardiac region (entire heart) is extracted and then a left ventricle region is extracted. In the present embodiment, each region is extracted by determining a contour of each region. More specifically, diagnosis assisting apparatus 1 calculates a characteristic amount representing likelihood of a contour of the heart and a characteristic amount representing a likelihood of a contour of the left ventricle with respect to a value of each voxel data constituting a three-dimensional anatomical image 7 and evaluates the calculated characteristic amounts based on an evaluation function obtained in advance by machine learning, thereby determining whether or not each voxel data represents a contour of the heart or a contour of the left ventricle (boundary between left ventricle and myocardium). By repeating the aforementioned determination, voxel data representing a contour of the entire heart and voxel data representing a contour of the left ventricle are extracted. In the present embodiment, Adaboost algorithm is used for obtaining the evaluation function. For more information with respect to the contour determination method, a reference is made, for example, to Japanese Unexamined Patent Publication No. 2007-307358. Note that other machine learning methods and statistical analysis methods, such as linear discriminant method, neural network, support vector machine, and the like may be used for the extraction of the cardiac region.

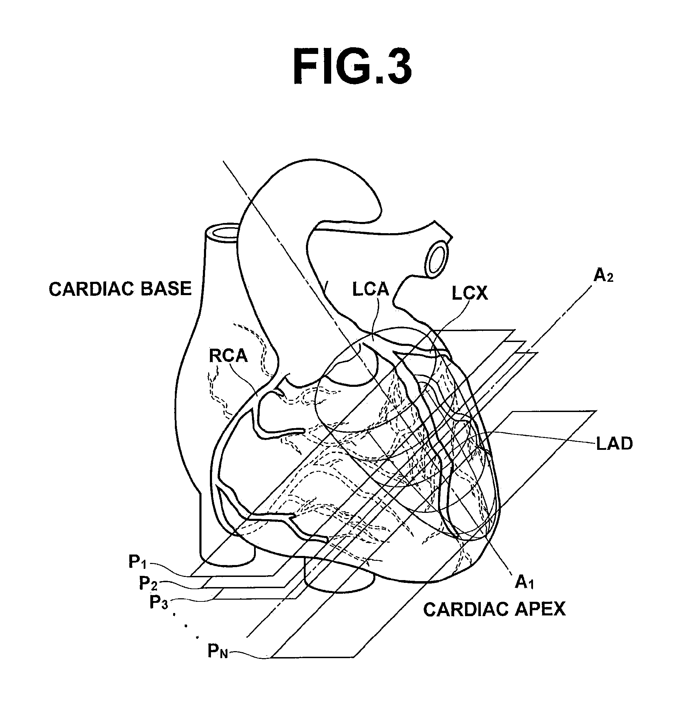

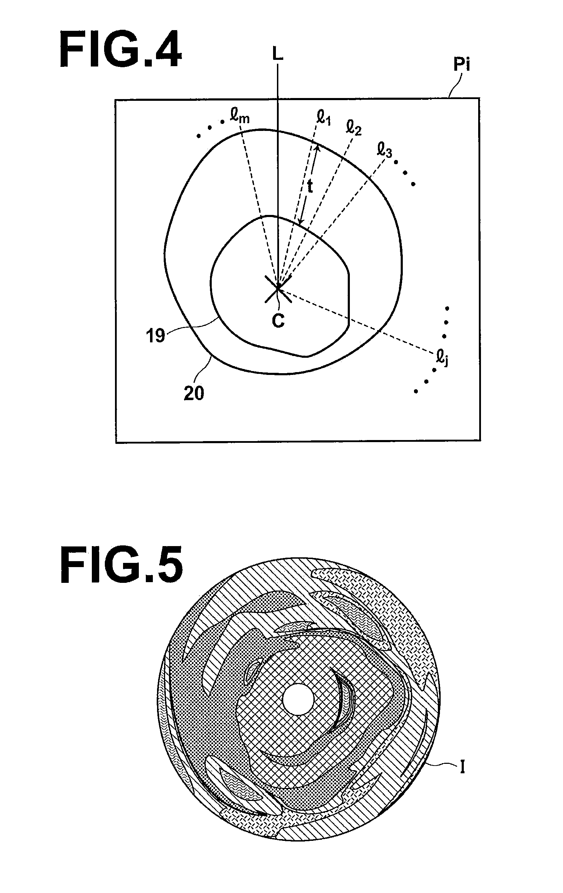

First index value calculation means 32 performs a cardiac function analysis process on the entire heart for the magnitude and direction of motion of each portion constituting the heart (S03) . Second index value calculation means 33 performs a cardiac function analysis process on a three-dimensional anatomical image 7 at a given phase or on a series of three-dimensional anatomical images 7 for calculating a second index value with respect to each portion constituting the heart. Figure 3 illustrates a long axis and cross-sections set in a cardiac function analysis process, Figure 4 illustrates an example evaluation value calculated in the cardiac function analysis process, and Figure 5 illustrates an example bull's eye image. The cardiac function analysis process will now be described in detail with reference to Figures 3 to 5.

As illustrated in Figure 3, in the cardiac function analysis process, first index value calculation means 32 and second index value calculation means 33 set a long axis A₁, connecting the cardiac apex, approximate center of the left ventricle, and cardiac base, and a short axis A₂ perpendicular to the long axis A₁ in the extracted left ventricle region. In the present embodiment, the long axis A₁ is set automatically by calculating positional coordinates of the cardiac apex and center of the left ventricle from the result of cardiac region extraction process. The short axis A₂ is set so as to pass through the center of the left ventricle and is perpendicular to the long axis A₁. The position or direction of the automatically set long axis, however, may be changed by a user operation. In the present embodiment, the automatically set long axis is displayed on a screen with a cardiac region image, and the position or direction of the long axis may be changed by a drag operation or a rotating operation.

Following this, first index value calculation means 32 and second index value calculation means 33 set a plurality of cross-sections P₁ to P_{N} which is perpendicular to the long axis. Then, in each cross-section P₁, a plurality of line segments l₁ to lₘ radially extending from an intersection point C of the long axis and the cross-section is defined, as illustrated in Figure 4. Then, coordinate values of a boundary 19 between the left ventricle and myocardium and an outer wall 20 of the myocardium are obtained on each line segment lᵢ and a myocardial wall thickness "t" is calculated from the coordinate values on each line segment lᵢ.

Further, first index value calculation means 32 may use various methods for analyzing the motion of a heart. As an example, a method that performs local template matching to track how each portion of the cardiac wall moves, as in the tissue tracking method, may be employed. Further, a method that measures how each portion of the cardiac wall moves by projecting a grid pattern at the time of imaging by a MRI system and measuring deformation of the grid pattern, as in the tagging method, may also be employed. In the present embodiment, for a plurality of time series three-dimensional anatomical images 7 at times T₁, T₂, ----- T_{Nt}, a process of analyzing motion of each portion of the cardiac wall is repeated by template matching between a three-dimensional anatomical image 7 at each time Tᵢ and a three-dimensional anatomical image 7 at time Tᵢ+ΔT (1≤ i ≤Nₜ), and each time an evaluation value representing a cardiac motion is calculated. In the case where there are Nₜ three-dimensional anatomical images for a heart for which three-dimensional anatomical images 7 of two or more phases are required for cardiac motion analysis, user may determine as to between which phases of three-dimensional anatomical images the cardiac motion is analyzed and may perform the aforementioned process.

Note that a method of generating a three-dimensional functional image 8 through analysis of the three-dimensional anatomical image 7 is detained, for example, in Japanese Unexamined Patent Publication No. 2002-306483 and Japanese Unexamined Patent Publication No. 2008-289799.

In the present embodiment, of those evaluation values calculated by a known method, an amount of cardiac wall motion in a direction perpendicular to the direction radially extending from the center of the heart to the outside of the heart is calculated as the first index value (S04), and an amount of cardiac wall motion in the direction radially extending from the center of the heart to the outside of the heart is calculated as the second index value (S05).

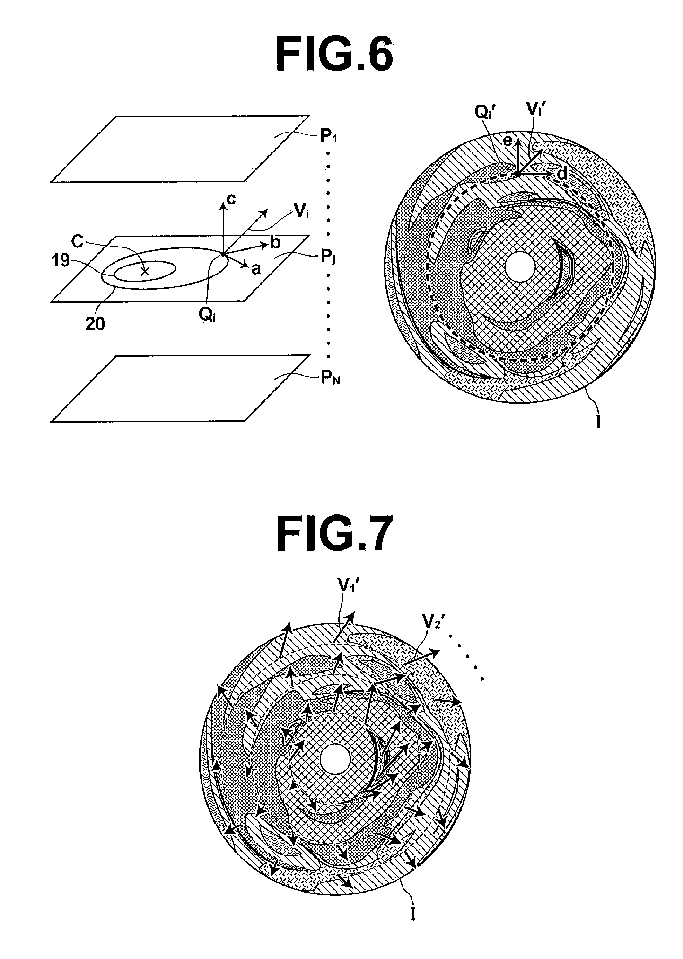

Figure 6 illustrates a calculation method of a first index value to be superimposed on a two-dimensional image of the present embodiment.

In the present embodiment, a comparison is made between a three-dimensional anatomical image at time T corresponding to a predetermined phase and a three-dimensional anatomical image at time T+ΔT corresponding to a further phase and the myocardium is divided into a plurality of portions Q₁, Q₂, -----, Qₘ (m is an integer not less than 2) with respect to each cross-section Pⱼ (1≤j≤N) of a plurality of cross-sections P₁ to P_{N} perpendicular to the set long axis.

Then, as illustrated in Figure 6, an evaluation value representing the motion of each divided myocardial portion Qi is calculated as a vector Vᵢ constituted by a component Vₐᵢ in "a" direction on the cross-section Pⱼ perpendicular to the direction from the intersection point C of the long axis and cross-section Pⱼ toward the myocardial portion Qᵢ, a component V_{bi} in "b" direction which is the direction from the intersection point C of the long axis and cross-section Pⱼ toward the myocardial portion Qᵢ, and a component V_{ci} in "c" direction perpendicular to the cross-section Pⱼ. The direction "a" on the cross-section Pⱼ perpendicular to the direction from the intersection point C of the long axis and cross-section Pⱼ toward the myocardial portion Qᵢ is roughly corresponds to a circumferential direction of the heart so that the component Vₐᵢ in the "a" direction on the cross-section Pⱼ roughly represents the motion of the heart in the circumferential direction on the cross-section Pⱼ.

Then, the component Vₐᵢ in the "a" direction on the cross-section Pⱼ and component V_{ci} in the "c" direction perpendicular to the cross-section Pⱼ of the vector Vᵢ are related respectively to a component V_{di} in a circumferential direction "d" and a component Vₑᵢ in a radial direction "e" of a corresponding portion Qᵢ' on a bull's eye image I to calculate a vector Vᵢ' constituted by the component V_{di} in the circumferential direction "d" and component Vₑᵢ in the radial direction "e" of the portion Qᵢ' and represents the first index value. In the present embodiment, the component V_{bi}, not included in the first index value, in the direction "b" extending along the cross-section from the intersection point of the long axis and cross-section is calculated as the second index value at the portion Qᵢ. Thereafter, the process of calculating the first and second index values is repeated for each myocardial portion Qᵢ (1≤ i ≤m) with respect to each cross-section Pⱼ (1≤j≤N) of the plurality of cross-sections Pᵢ to P_{N} perpendicular to the set long axis and whereby first and second index values are calculated for the entire heart.

In the case where the volume data for calculating the first index value and the volume data for calculating the second index value are the same, as in the present embodiment, positional alignment of myocardial portion Qᵢ is not required. In the case where the volume data for calculating the first index value and the volume data for calculating the second index value are different, the positional alignment of corresponding portions between the different volume data may be performed by various known methods, such as those described, for example, in Japanese Unexamined Patent Publication Nos. 2008-289799 and 2008-253753.

The first index value calculation process may be performed first or the second index value calculation process may be performed first, otherwise they may be performed simultaneously. Further, for an overlapping process between the first index value calculation process and the second index value calculation process, either one of the processing result may be utilized by the other.

In the case where second index value calculation means 33 does not use the processing result of the first index value calculation process for the second index value calculation process, such as the case where a membrane thickness variance is used as the second index value, the second index value calculation means 33 separately calculates an evaluation value for calculating the second index value by a known method, such as a method that obtains a difference in coordinate values between three-dimensional anatomical images 7 of different phases. This process may yield a three-dimensional functional image 8 in which evaluation values are disposed according to the shape of the heart. The generated three-dimensional functional image 8 is stored in memory 3. Further, second index value calculation means 33 may be a means that calculates a plurality of types of evaluation values and generates a three-dimensional functional image 8 in which evaluation values are disposed according to the shape of the heart with respect to each of the plurality of types of evaluation values.

As in the present embodiment, not only the first and second index values are obtained from a three-dimensional anatomical image but image obtaining means 31 further obtains a three-dimensional functional image 8 in which evaluation values for evaluating the cardiac function are disposed three-dimensionally according to the shape of the organ. Here, second index value calculation means 33 may be a means that calculates the second index value that serves as a diagnostic indicator from the three-dimensional functional image instead of at least either one of a three-dimensional anatomical image at a given phase and a three-dimensional anatomical image group, and two-dimensional image generation means 34 may be a means that generates a two-dimensional image based on the calculated second index value.

For example, where a three-dimensional functional image 8, such as a SPECT image or the like, is stored in memory 3, second index value calculation means 33 may use the data constituting the three-dimensional functional image 8 as the second index value without performing the cardiac function analysis process.

Evaluation values represented by a three-dimensional functional image 8 include, for example, an evaluation value that can be calculated from a single three-dimensional anatomical image 7, such as the wall thickness. The evaluation values may further include those calculated by analyzing a plurality of time series three-dimensional anatomical images 7, such as the amount of wall movement, wall thickness variance, ejection fraction, and the like. The further obtained three-dimensional functional image 8 may be an image obtained as a result of a cardiac function analysis on a three-dimensional anatomical image 7, an image outputted from an imaging system for generating a functional image, such as a SPECT image or the like, or an image obtained by evaluation values which combine evaluation values outputted from an imaging system for generating a functional image, such as a SPECT image or the like, and evaluation values obtained from a three-dimensional anatomical image 7.

Next, two-dimensional image generation means 34 generates a two-dimensional image of the entire heart in which the second index value of each cardiac portion is disposed in a planar fashion. Here, a bull' s eye image I is generatedbasedon second index values (SO6) . .In the bull's eye generation process, diagnosis assisting apparatus 1 generates a bull's eye image I by arranging second index values included in cross-sections P₁ to P_{N} of a three-dimensional functional image 8 on circumferences of concentric circles having different radii with respect to each cross-section. In the present embodiment, a bull's eye image I in which evaluation values of cross-section P_{N} closest to the cardiac apex are disposed on a circle having the smallest radius and evaluation values of cross-section P₁ most remote from the cardiac apex are disposed on a circle having the largest radius, with the cardiac apex as the center of concentric circles, is generated. An example bull's eye image I generated by such processing is shown in Figure 5.

Then, display control means 35 displays the generated two-dimensional image on display 5 and superimposes marks representing the first index values calculated by the index value calculation means on the displayed two-dimensional image in a manner that allows the direction of motion of the first index values to be visually recognized (S07).

Figure 7 shows an example bull's eye image I in which arrows representing vectors V₁' to V_{N}' which, in turn, represent first index values are displayed on a plurality of points disposed at a predetermined interval on circumferences of concentric circles having different radii with respect to each cross-section. As shown in Figure 7, in the present embodiment, display control means 35 superimposes an arrow representing a vector Vᵢ' having a component V_{di} in the circumferential direction and a component Vₑᵢ in the radial direction on the bull' s eye image I as the first index value at each myocardial portion.

According to the present embodiment, marks representing first index values are superimposed on a two-dimensional image of the entire heart in which a second index value of each cardiac portion is disposed in a planar fashion in a manner that allows the direction of motion of the first index values to be visually recognized. This allows the index values that serve as diagnostic indicators of the entire heart and direction of motion of each cardiac portion to be displayed simultaneously on the displayed image. This allows the doctor or radiologist to confirm both the index values serving as diagnostic indicators of the entire heart and direction of motion of each cardiac portion on the displayed image, whereby the index values serving as diagnostic indicators of the entire heart and direction of motion of each cardiac portion may be easily understood. Consequently, motion of the cardiac wall may be understood easily without requiring any further processing, such as rotating the image or the like. In addition, first index values may be comprehended together with second index values represented by the two-dimensional image, so that an abnormality of cardiac function and the like may be estimated easily.

Further, in the present embodiment, display control means 35 displays marks representing first index values at a plurality of points regularly disposed in a two-dimensional image. This allows index values serving as diagnostic indicators of the entire heart and direction of motion of each cardiac portion to be displayed simultaneously on the displayed image and the direction of motion of each cardiac portion to be understood more easily.

Further, as arrows are used by display control means 35 as the marks representing first index values, the direction of motion of each cardiac portion may be understood further easily.

As two-dimensional image generation means 34 of the diagnosis assisting apparatus of the present invention is a means that generates a bull's eye image I as the two-dimensional image, evaluation values serving as diagnostic indicators of the entire heart and direction of motion of each cardiac portion may be displayed simultaneously in the displayed bull's eye image I. This allows a doctor or a radiologist to confirm both the index values serving as diagnostic indicators of the entire heart and direction of motion of each cardiac portion in the displayed bull's eye image, whereby the index values serving as diagnostic indicators of the entire heart and direction of motion of each cardiac portion may be easily understood.

Hereinafter, other examples of first index values and second index values to which the diagnosis assisting method of the present invention is applicable will be described.

The first index value may include any of all types of index values representing a direction and magnitude of each region of a cardiac wall between a three-dimensional image 7 at a given phase and a three-dimensional image 7 at a further phase. Further, the first index value may be a value of data constituting a three-dimensional functional image 8 or a bull's eye image I as long as it represents a direction and magnitude of the motion of each region of a cardiac wall or a value obtained as a result of certain analysis performed on the value of data. The term "certain analysis" as used herein may include, for example, obtaining an average value (including weighted average or the like), a maximum value, or a minimum value of each data of movement speed and acceleration of the cardiac wall between specific phases, such as from the end-diastole to the end-systole.

For example, it is conceivable that the following index values of evaluation values constituting a three-dimensional functional image 8 or a bull's eye image I can be calculated and presented as the first index values:
(i) amount of wall movement: value indicating displacement of wall position between end-diastole and end-systole of the heart;
(ii) cardiac wall movement speed; and
(iii) acceleration of cardiac wall: value indicating variance in cardiac wall movement speed.

The second index value may be anything as long as it is an index value useful for diagnosis, and it may be a value of data constituting a three-dimensional functional image 8 or a bull' s eye image I, or a value obtained as a result of certain analysis performed on the value of data. For example, it may be a value obtained by the process of averaging (including weighted average or the like) each data constituting a three-dimensional anatomical image 7 or a three-dimensional functional image 8, or a value obtained by the process of obtaining a maximum or minimum value of the data. Further, the second index value may be the same index value as the first index value or a different index value from the first index value.

For example, it is conceivable that the following index values of evaluation values constituting a three-dimensional functional image 8 or a bull's eye image I can be calculated and presented as the second index values:
(iv) amount of wall movement: a value indicating displacement of wall position between end-diastole and end-systole of the heart;
(v) wall thickness variance: difference in thickness of myocardium between diastolic phase and systolic phase;
(vi)wall thickness growth rate: value indicating the ratio between wall thickness variance and wall thickness in diastolic phase;
(vii) amount of wall motion: value indicating difference in cardiac ventricle diameter between diastolic phase and systolic phase; and
(viii) ejection fraction: value indicating ratio between blood volume pumped out by a single contraction and heart volume in diastolic phase.

In the aforementioned embodiment, cardiac motion on each plane in the direction "a" perpendicular to the direction from the long axis of the heart toward each myocardial portion Qᵢ of each cross-section may be displayed as one of the first index values representing each myocardial portion Qᵢ of each cross-section, and cardiac motion in the direction "b" from the long axis of the heart toward each myocardial portion Qᵢ may be displayed as the second index value. This allows a twisting motion of the heart to be displayed in an easily understandable manner on the displayed image, so that the doctor or the like may easily understand the twisting motion of the myocardium of the entire heart. Whereas, conventional methods can display only an index value in the radial direction in a two-dimensional image, the present embodiment may display motion of each cardiac portion in three directions on a two-dimensional image by the first index value, whereby the cardiac motion may be displayed in detail and accurately.

Further, as a modification of the present embodiment, the component Va in the direction "a" on a cross-section Pⱼ perpendicular to the direction "b" from the long axis of the heart to each myocardial portion Qᵢ of each cross-section, which is one of the first index values representing each myocardial portion Qᵢ of each cross-section, may be related to the component V_{di} in the circumferential direction "d" in the bull's eye image I, as illustrated in Figure 6. Then, display control means 35 may determine the magnitude of the component Vₐᵢ in the circumferential direction as the length of the arrow. In this way, in the case where the myocardial motion on each plane perpendicular to the direction "b" from the long axis of the heart to each myocardial portion Qᵢ of each cross-section is displayed as the first index value, and myocardial motion in the direction "b" from the long axis of the heart toward each myocardial portion Qᵢ is displayed as the second index value. This allows cardiac motion in the two directions to be displayed in an easily understandable manner on the displayed image, so that the doctor or the like may easily understand the twisting motion of the myocardium of the entire heart in the circumferential direction.

Further, with respect to the movement of each myocardial portion Qᵢ of each cross-section, the component Vₐ on each cross-section Pⱼ in a direction perpendicular to the direction "b" from the long axis of the heart toward each myocardial portion Qᵢ and the component V_{b} in the direction "b" from the long axis of the heart to each myocardial portion Qᵢ may be related to the component V_{di} in the circumferential direction "d" of the bull's eye image I and component Vₑᵢ in the radial direction of the bull's eye image I respectively. Then, display control means 35 may display each vector Vᵢ' which includes the component V_{di} in the circumferential direction and the component Vₑᵢ in the radial direction. As described above, in the case where the first index value represents the cardiac motion in a direction perpendicular to the direction "b" from the long axis of the heart toward each myocardial portion Qᵢ on the displayed image, the doctor or the like may easily understand. twisting motion of the myocardium of the entire heart. Further, in the case where both the first and second index values represent cardiac motion, the cardiac motion may be represented by a plurality of different representation methods, thereby allowing the doctor or the like to understand the cardiac motion in more detail.

Further, in the present embodiment, display control means 35 may display the arrow such that the greater the magnitude of the first index value, the wider the arrow. This allows the user to intuitively understand the magnitude of the first index value.

Still further, in the aforementioned example, the magnitude of a component in a direction not included in the vector V' or the magnitude of a vector formed by adding up each component, including a component in a direction not included in the vector V' , as auxiliary information. For example, the auxiliary information may be numerically displayed adjacent to the arrow, or the magnitude of a component in a direction not included in the vector V' or the magnitude of a vector formed by adding up each component, including a component in a direction not included in the vector V', may be related to arrows having different widths, colors, shapes of arrow head or arrow feather in advance with respect to each range of the magnitudes, and the auxiliary information may be displayed in a visually recognizable manner by the width, color, shape of arrow head or arrow feather of each arrow. Still further, a different color may be disposed and displayed on each point of the mark according to the value of the first index.

Further, the display method is not limited to this, and marks representing first index values maybe displayed in different colors with respect to direction or magnitude of motion of each cardiac portion by various methods according to the user requirements.

Further, as a modification of the present embodiment, image obtaining means 31 may be a means that further obtains a three-dimensional functional image 8 in which evaluation values for evaluating the cardiac function are disposed three-dimensionally according to the shape of the organ, and second index value calculation means 33 may be a means that calculates the second index value that serves as a diagnostic indicator from the three-dimensional functional image instead of at least either one of the three-dimensional anatomical image at a given phase and the three-dimensional anatomical image group. In this case, index values for evaluating cardiac function and the direction of motion of each cardiac portion for the entire heart may be displayed simultaneously on the displayed image. This allows the doctor or radiologist to confirm both the index values for evaluating the cardiac function and direction of motion of each cardiac portion on the displayed image and to easily understand the evaluation values for evaluating the cardiac function and direction of each cardiac portion for the entire heart.

In the present embodiment, two-dimensional image generation means 34 generates a two-dimensional image to which first index values are related, and display control means 35 displays the two-dimensional image on display 5, whereby marks representing the first index values are superimposed on the two-dimensional image in a manner that allows the direction of motion of each first index value to be visually recognized. But the method is not limited to this, and a method that separately generates a two-dimensional image based on first index values and a two-dimensional image based on second index values and displays the images in a superimposing manner if the method is capable of superimposing the generated two-dimensional image and marks representing first index values in a manner that allows the direction of motion of each first index value to be visually recognized.

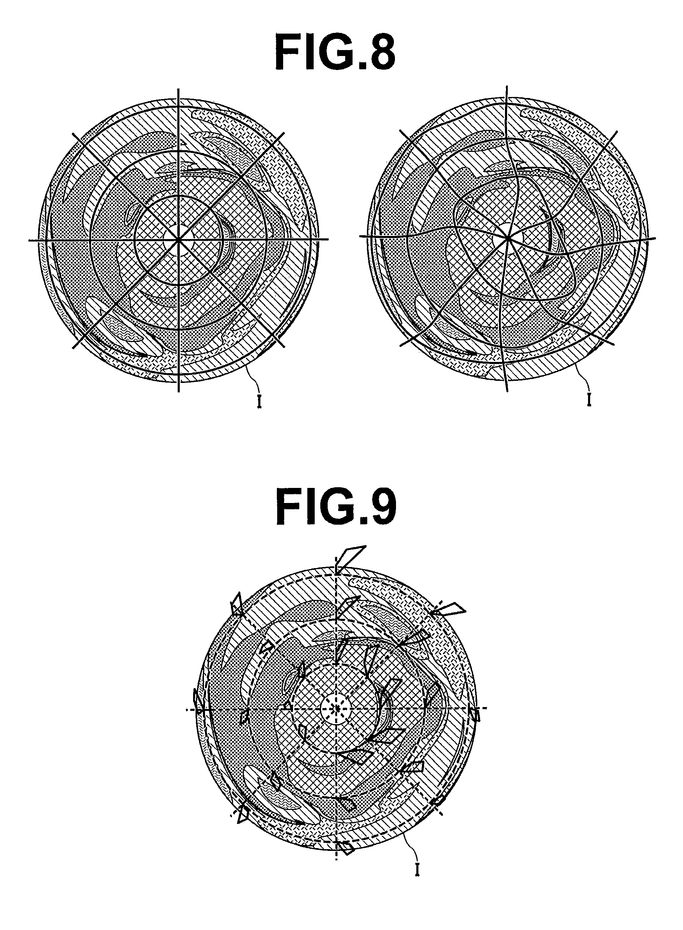

Further, various types of marks may be used as the mark representing the first index as long as it may represent a direction and magnitude of the cardiac motion. For example, various types of marks capable of representing a vector may be used and not limited to the arrow. Figures 8 and 9 illustrate examples of different marks representing first index values. Figure 8 is an example bull's eye image I on which a grid representing the first index value of the present embodiment is superimposed, and Figure 9 is an example Bull's eye image I on which a trajectory representing the first index value of the present embodiment is superimposed.

As illustrated in Figure 8, display control means 35 may shift each of a plurality of points disposed on a two-dimensional image so as to form a regular grid, as shown in the drawing on the left of Figure 8, to a position based the magnitude and direction of the first index value at each position, as shown in the drawing on the right of Figure 8, and display the shifted positions as the marks representing the first index values. In this case, the direction of motion of each cardiac portion may be easily understood.

Further, marks, each representing the first index value, may be displayed on a two-dimensional image in combination with each other. For example, the grid shown on the right of Figure 8 and the arrows shown in Figure 7 may be displayed in a bull's eye image. Use of a plurality of marks allows the cardiac motion to be displayed by various representation methods, leading to better user understanding.

Still further, display control means 35 may display trajectories of a plurality of points in a two-dimensional image due to changes in cardiac phase, as shown in Figure 9. Figure 9 is an imaginary drawing indicating a trajectory of each of a plurality of points disposed at a predetermined interval in concentric circles having different radii in a bull's eye image I due to changes in the phase from the end-diastole to end-systole. In this case, the direction of motion of each cardiac portion may be understood easily. Further, each trajectory may be gradationally displayed according to the speed, acceleration, or the like in each phase on the trajectory, for example, such that the greater the speed or acceleration, the greater the color density of the trajectory. In this case, not only the movement of each position according to the phase changes but also other motions such as the speed, acceleration, and the like may be displayed, so that cardiac motion can be understood in more detail.

In the first embodiment, first index value calculation means 32 calculates, with respect to Nₜ three-dimensional anatomical images 7, first index values from each three-dimensional anatomical image 7 and a three-dimensional anatomical image 7 in the successive phase of each three-dimensional anatomical image 7. Preferably, two-dimensional image generation means 34 is a means that generates a two-dimensional image to which first index values in each phase are related in a time series manner and display control means 35 is a means that displays the marks at each phase in a time series manner.

In this case, the cardiac motion that varies with each phase can be understood easily as well as the index values that serve as diagnostic indicators for the entire heart and direction of motion of each cardiac portion may be displayed simultaneously on the displayed image.

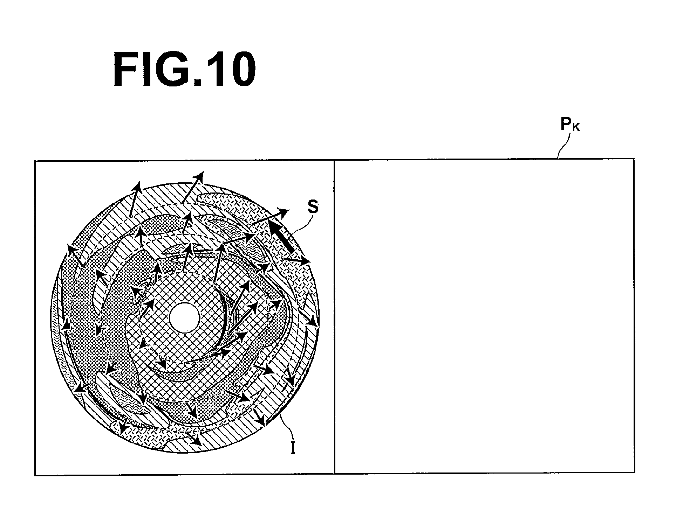

Figure 10 illustrates an example modification of the first embodiment. The diagnosis assisting apparatus of the present embodiment may accept specification of a mark superimposed on a bull's eye image I and display a tomographic image at the position corresponding to the specified mark. For example, as shown in Figure 10, the user may move the cursor S and click on an arbitrary position of an arrow shown in the bull' s eye image I with a mouse or the like and the apparatus may display a tomographic image Pₖ which includes the position corresponding to the position of the base of the clicked arrow.

Further, in the aforementioned case, a position in the length direction of an arrow in the bull's eye image I may be related to a predetermined time along the phase change. For example, each position in the length direction of an arrow may be related to each phase along a predetermined temporal progress such that the base of the arrow corresponds to the end-diastole and the arrow head corresponds to the end-systole. Then, when an arbitrary position of an arrow is clicked by the user with a mouse or the like, a tomographic image of a three-dimensional anatomical image at the phase corresponding to the position of the clicked arrow and includes the position corresponding to the position of the arrow base is displayed and an image corresponding to a tomographic image which includes the position corresponding to the position of the base of the arrow in the successive phase is displayed as a motion image in a time series manner.

In this case, when an arbitrary position of an arrow is clicked by the user with a mouse or the like and the mouse is dragged from the clicked position, the time series motion image display period may be determined according to the dragged distance or time. That is, the ratio of reproduction time to a predetermined time is related to each dragged distance or time in advance and the tomographic image which includes the position corresponding to the position of the arrow base is displayed by the time related to the dragged distance or time from the phase corresponding to the position of the clicked arrow, and the image corresponding to the tomographic image which includes the position of the arrow base in the successive phase is displayed as a motion image in a time series manner.

Diagnosis assisting apparatus 1 may further includes a coronary artery extraction means to extract a coronary artery by one of the various known methods and superimpose the extracted coronary artery region on the screen of the display device by any known method of displaying a coronary artery on a bull' s eye image I in a superimposing manner as described, for example, in Japanese Unexamined Patent Publication Nos. 2005-027999 and 2008-253753. In this case, it is preferable that diagnosis assisting apparatus 1 is configured to allow the user to correct the route of the displayed coronary artery or to manually set a portion of the route not extracted by the automatic extraction on the screen. That is, it is preferable that diagnosis assisting apparatus 1 is capable of detecting a user operation on the screen, correcting information of the coronary artery stored in the memory based on the user operation, and updating the coronary artery region displayed on the screen based on the corrected information.

In the present embodiment, the diagnosis assisting apparatus is configured to control the type of mark displayed on the screen and display timing (display switching) by referring to mark setting information stored in memory 3 or by detecting menu selection operation by the user.

For example, the display control means allows a mark representing the first index to be selectable through a GUI or the like and detects a user selection operation to perform the following display operations. That is, when an arrow is specified as the mark of the first index, the display control means displays first index values by arrows on a bull' s eye image I in a superimposing manner, when a trajectory of each portion is specified as the mark of the first index, the display control means displays first index values by the trajectory of each point on the bull's eye image I in a superimposing manner, and when a grid pattern is specified as the mark of the first index, the display control means displays a pattern formed by shifting a plurality of points disposed so as to form a regular grid based on the first index values on the bull's eye image I in a superimposing manner. As to whether or not to display the marks representing first index values, it is preferable that the bull's eye image I on which the marks representing first index values are superimposed, as illustrated in Figure 7 by way of example, and the bull's eye image I, as illustrated in Figure 5 by way of example, are stored separately and switchably displayed according to the request from the user. Diagnosis assisting apparatus 1 determines whether or not to perform a superimposing display on a bull's eye image I and with which mark the superimposing display is performed according to information set in the apparatus or user operation.

In diagnosis assisting apparatus 1, second index value calculation means 33 may be a means that calculates different types of second index values, two-dimensional image generation means 34 may be a means that generates a two-dimensional image with respect to each of different types of second index values, and display control means 35 may be a means that displays the marks representing first index values on a two-dimensional image desired by the user in a superimposing manner.

Further, in each embodiment described above, a bull's eye image I is generated such that values obtained from the cross-section P_{N} closest to the cardiac apex are disposed on a circle having a smallest radius and values obtained from the cross-section Pᵢ most remote from the cardiac apex are disposed on a circle having a largest radius, with the cardiac apex as the center of the concentric circles. But the bull's eye may be generated such that values obtained from the cross-section Pᵢ are disposed on a circle having a smallest radius and values obtained from the cross-section P_{N} are disposed on a circle having a largest radius, with the cardiac base as the center of the concentric circles. For the bull's eye image, various other modifications are known and any of the methods may be used in the present invention.

Still further, in each embodiment described above, diagnosis assisting apparatus 1 is formed of a single computer on which each program is installed. But a diagnosis assisting system that can realize functions identical to those of diagnosis assisting apparatus 1 may be constructed by distributing and installing respective programs on a plurality of computers.

In each embodiment described above, diagnosis assisting apparatus 1 may include a means for performing print output or data output (recording on a medium, such as .CD-R or DVD, or transfer via network) other than display output. That is, in the present invention, the output form of index values is not limited to the display output.

As described above, the present invention is not limited to the embodiments described above, and various changes and modifications may be made without departing from the spirit of the present invention.

## Claims

1. A diagnosis assisting apparatus, comprising:
an image obtaining means (31) for obtaining a three-dimensional anatomical image group that includes three-dimensional anatomical images (7), each three-dimensionally representing a structure of a heart, obtained by imaging the heart at each phase;
a first index value calculation means (32) for calculating a first index value representing a direction and magnitude of motion of each portion of the heart for a three-dimensional anatomical image (7) at a given phase of the three-dimensional anatomical image group with respect to a further phase;
a second index value calculation means (33) for calculating a second index value that serves as a diagnostic indicator of each portion of the heart from the three-dimensional anatomical image (7) at the given phase or the three-dimensional anatomical image group;
a two-dimensional image generation means (34) for generating a two-dimensional image (I) of the entire heart in which the second index value of each portion of the heart is disposed in a planar fashion; and
a display control means (35) for displaying the generated two-dimensional image on a display device (5) and superimposing a mark (Vᵢ') representing the first index value calculated by the first index value calculation means (32) on the displayed two-dimensional image in a manner that allows the direction of motion of the first index value to be visually recognized.

2. The apparatus of claim 1, **characterized in that**:
the image obtaining means (31) is a means that further obtains a three-dimensional functional image (8) in which evaluation values for evaluating function of the heart are disposed three-dimensionally according to the shape of the heart; and
the second index value calculation means (33) is a means that calculates the second index value that serves as a diagnostic indicator from the three-dimensional functional image (8) instead of at least either one of the three-dimensional anatomical image (7) at the given phase and the three-dimensional anatomical image group.

3. The apparatus of claim 1 or 2, **characterized in that**:
the first index value calculation means (32) is a means that calculates the first index value for three-dimensional anatomical images (7) at a plurality of phases of the heart; and
the display control means (35) is a means that displays the mark (Vᵢ') at each of the plurality of phases in a time series manner.

4. The apparatus of any of claims 1 to 3, **characterized in that** the display control means (35) is a means that displays the mark (Vᵢ') representing the first index value at each of a plurality of points disposed regularly in the two-dimensional image (I).

5. The apparatus of claim 4, **characterized in that** the display control means (35) is a means that uses an arrow as the mark (Vᵢ') representing the first index value.

6. The apparatus of claim 4, **characterized in that** the display control means (35) is a means that displays a trajectory of each of the plurality of points in the two-dimensional image (I) due to a change in phase of the heart as the mark (Vᵢ') representing the first index value.

7. The apparatus of any of claims 1 to 3, **characterized in that** the display control means (35) is a means that uses each of a plurality of points disposed in the two-dimensional image so as to form a regular grid and shifted based on a magnitude and direction of the first index value at each point as the mark (Vᵢ') representing the first index value.

8. The apparatus of any of claims 1 to 7, **characterized in that** the two-dimensional image generation means (34) is a means that generates a bull's eye image.

9. A computer-implemented diagnosis assisting method, comprising the steps of:
obtaining a three-dimensional anatomical image group that includes three-dimensional anatomical images (7), each three-dimensionally representing a structure of a heart, obtained by imaging the heart at each phase;
calculating a first index value representing a direction and magnitude of motion of each portion of the heart for a three-dimensional anatomical image (7) at a given phase of the three-dimensional anatomical image group with respect to a further phase;
calculating a second index value that serves as a diagnostic indicator of each portion of the heart from the three-dimensional anatomical image (7) at the given phase or the three-dimensional anatomical image group;
generating a two-dimensional image (I) of the entire heart in which the second index value of each portion of the heart is disposed in a planar fashion; and
displaying the generated two-dimensional image (I) on a display device (5) and superimposing a mark (Vᵢ') representing the first index value calculated by the first index value calculation means (32) on the displayed two-dimensional image (I) in a manner that allows the direction of motion of the first index value to be visually recognized.

10. A computer readable recording medium on which is recorded a diagnosis assisting program for causing a computer to function as:
an image obtaining means (31) for obtaining a three-dimensional anatomical image group that includes three-dimensional anatomical images (7), each three-dimensionally representing a structure of a heart, obtained by imaging the heart at each phase;
a first index value calculation means (32) for calculating a first index value representing a direction and magnitude of motion of each portion of the heart for a three-dimensional anatomical image (7) at a given phase of the three-dimensional anatomical image group with respect to a further phase;
a second index value calculation means (33) for calculating a second index value that serves as a diagnostic indicator of each portion of the heart from the three-dimensional anatomical image (7) at the given phase or the three-dimensional anatomical image group;
a two-dimensional image generation means (34) for generating a two-dimensional image (I) of the entire heart in which the second index value of each portion of the heart is disposed in a planar fashion; and
a display control means (35) for displaying the generated two-dimensional image on a display device (5) and superimposing a mark (Vᵢ') representing the first index value calculated by the first index value calculation means (32) on the displayed two-dimensional image in a manner that allows the direction of motion of the first index value to be visually recognized.
